(19) Europäisches Patentamt
European Patent Office
Office européen des brevets

(11) **EP 3 120 768 A1**

(12) **EUROPEAN PATENT APPLICATION**

(43) Date of publication:
**25.01.2017 Bulletin 2017/04**

(51) Int Cl.:
**A61B 5/145** (2006.01)   **A61B 5/1455** (2006.01)

(21) Application number: **16178509.2**

(22) Date of filing: **07.07.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **21.07.2015 JP 2015144013**

(71) Applicant: **Fuji Xerox Co., Ltd.**
**Minato-ku,**
**Tokyo (JP)**

(72) Inventors:
• **MATSUSHITA, Kazuyuki**
**Ebina-shi, Kanagawa (JP)**
• **TAKEDA, Kazutaka**
**Ebina-shi, Kanagawa (JP)**
• **YUKAWA, Kohei**
**Ebina-shi, Kanagawa (JP)**
• **OZAWA, Hideaki**
**Ebina-shi, Kanagawa (JP)**
• **KINOSHITA, Taku**
**Ebina-shi, Kanagawa (JP)**
• **NAKAYAMA, Hideo**
**Ebina-shi, Kanagawa (JP)**

(74) Representative: **Ward, James Norman**
**Haseltine Lake LLP**
**Bürkleinstrasse 10**
**80538 München (DE)**

(54) **CONCENTRATION CALCULATION SYSTEM OF OPTICALLY ACTIVE SUBSTANCE AND PROGRAM**

(57)    A concentration calculation system of an optically active substance, includes: a calculation unit configured to: acquire an amount of change in a polarization state by allowing light having different wavelengths to pass through a cornea and an aqueous humor; and calculate a concentration of a specific optically active substance contained in the aqueous humor by a least squares method based on a theoretical formula which includes a matrix representing a polarization property of the cornea and a matrix representing a polarization property of the aqueous humor and represents a wavelength dependence of the amount of the change, wherein the matrix representing the polarization property of the aqueous humor is represented by a function of an expression representing the wavelength dependence of an optical rotation degree of the specific substance and the expression includes a concentration value of the specific substance as an unknown quantity or a temporal known quantity.

FIG. 2

EP 3 120 768 A1

**Description**

(i) Technical Field

The present invention relates to a concentration calculation system of an optically active substance and program.

(ii) Related Art

Patent Document 1 discloses a birefringence characteristics measurement method in which a sample, a 1/4 wavelength plate of which a principal azimuth is coincident with a polarization azimuth of a linear polarization for measurement, a linear polarizer (analyzer) provided with a polarization axis azimuth which is orthogonal to the polarization azimuth of the linear polarization for measurement, and a photo-detector are arranged on an emitting optical light axis of linear polarization for measurement, an actual measurement of an emission intensity of emitting light is performed by detecting light, which is emitted from the linear polarizer when the sample is relatively rotated with respect to the polarization axis azimuth of the linear polarization for measurement, the 1/4 wavelength plate, and the linear polarizer around the emitting optical axis, by the photo-detector, and a coefficient for calculating the birefringence azimuth and the birefringence amount of the sample is obtained by fitting a least squares method to a predetermined equation, in obtaining a birefringence azimuth and a birefringence amount of the sample.

Patent Document 2 discloses an optical characteristics measurement apparatus including an optical system, a light intensity information acquisition unit that acquires light intensity information of measuring light, and an operation processing unit. The optical system is configured in such a way that light emitted from a light source is made to incident onto a sample through a polarizer, an 1/2 wavelength plate, and an 1/4 wavelength plate, and light modulated by the sample is made to incident onto a light receiving unit through an analyzer. The light intensity information acquisition unit acquires light intensity information of plural measuring light. The operation processing unit performs operation processing for calculating at least one of a birefringence phase difference, a principal azimuth, and an optical rotation angle of the sample on the basis of a theoretical formula of light intensity of measuring light and the light intensity information of the measuring light.

Patent Document 3 discloses an optical anisotropy evaluation method in which a magnitude and a direction of optical anisotropy of the sample are evaluated on the basis of processing for obtaining an amplitude and a phase of at least one frequency component of a component of a frequency $2F (A_A - A_P + A_R)$ and a component of a frequency $2F (A_A + A_P - A_R)$ from plural light intensities $I (t_i)$ corresponding to plural times $t_i$, processing for obtaining two amplitudes and two phases of at least two frequency components of a component of a frequency $2F (A_A - A_P)$, a component of a frequency $2F (A_A + A_P)$, a component of a frequency $2F (A_A + A_P - 2A_R)$, and a component of a frequency $2F (A_A - A_P + 2A_R)$ from plural light intensities $I(t_i)$, and the amplitude and the phase obtained by the processing, in a case where a light intensity $I_i$ detected by a detector is acquired, a numerical progression $\{t_i\}$ is regarded as a time row obtained by lining up elapsed times $t_i$ from a reference time, the obtained numerical progression $\{I_i\}$ is regarded as a light intensity $I(t_i)$ detected by a detector in the elapsed times $t_i$ from reference time, and F is set to a predetermined coefficient (unit is angle$^{-1}$) when angles of a polarizer, an analyzer, and a phase difference plate are respectively set to $\phi_{P,i}$, $\phi_{A,i}$, $\phi_{R,i}$, that are designated by i.

Patent Document 4 discloses an optical rotation measurement method in a blood sugar level measurement apparatus. In the method, an orthogonal separation unit divides transmission light, which is resulted from transmitting though a subject of the linear polarization, into lights orthogonal to each other, a light detection unit receives the separated lights orthogonal to each other, a specification calculation unit calculates a specification of an optical system using a sum of signals detected by the light detection unit and a difference between the signals in a case where existing material is used as the subject, and an optical rotation calculation unit calculates optical rotation degree of a measuring target using the specification of the optical system and the signal detected by the light detection unit in a case where the subject is used as the measuring target.

Patent Document 5 discloses an optical characteristics measurement apparatus including an optical system, a light intensity information acquisition unit that acquires a piece of light intensity information of measuring light, and an operation processing unit. The optical system is configured in such a way that light emitted from a light source is made to incident onto the sample through a polarizer, a 1/2 wavelength plate, and a first 1/4 wavelength plate, and light emitted from the sample is made to incident onto a light receiving unit through a second 1/4 wavelength plate, and an analyzer and also in such a way that the 1/2 wavelength plate, the first 1/4 wavelength plate and the second 1/4 wavelength plate, and the analyzer are rotatable. The light intensity information acquisition unit acquires the piece of light intensity information of plural measuring light. The operation processing unit calculates at least matrix elements of a matrix that represents optical characteristics of the sample on the basis of a theoretical formula of light intensity of measuring light and the piece of light intensity information of the measuring light.

[Patent Document 1] JP-A-2008-032594
[Patent Document 2] JP-A-2007-139722
[Patent Document 3] JP-A-2012-103222
[Patent Document 4] JP-A-2014-130045
[Patent Document 5] Pamphlet of International Publication No. 2007/111159

SUMMARY

In a configuration in which light is emitted to an anterior eye chamber of an eyeball of a subject (testee) and a concentration of optically active substances contained in the aqueous humor is measured on the basis of a polarization state of light which passes through the anterior eye chamber, the polarization state of light is changed by the cornea present in a path of light and thus, it is difficult to calculate the concentration of the optically active substances contained in the aqueous humor with desired accuracy.

An object of the present invention is to accurately measure the concentration of optically active substances contained in the aqueous humor compared to a case where influence by the cornea is not taken into account.

A first aspect of the invention is a concentration calculation system of an optically active substance, comprising: a calculation unit configured to: acquire an amount of change in a polarization state by allowing light having different wavelengths to pass through a cornea and an aqueous humor; and calculate a concentration of a specific optically active substance contained in the aqueous humor by a least squares method based on a theoretical formula which includes a matrix representing a polarization property of the cornea and a matrix representing a polarization property of the aqueous humor and represents a wavelength dependence of the amount of change in the polarization state, wherein the matrix representing the polarization property of the aqueous humor is represented by a function of an expression representing the wavelength dependence of an optical rotation degree of the specific optically active substance and the expression includes a concentration value of the specific optically active substance as an unknown quantity or a temporal known quantity.

A second aspect of the invention is the concentration calculation system of an optically active substance according to the first aspect, wherein the calculation unit is configured to calculate the concentration of the specific optically active substance by: setting the value of the concentration of the specific optically active substance contained in the aqueous humor to the temporary known quantity and obtaining an initial value adjusted with regard to the cornea by the least squares method such that an objective function, that is a sum of squares of a difference between the acquired amount of change in the polarization state and the theoretical formula becomes smaller than a case where other initial values are used for the objective function; and calculating values allocated to the unknown quantities related to the aqueous humor and the cornea in the least squares method using the adjusted initial value such that the objective function becomes smaller than a case where other values are allocated for the objective function.

A third aspect of the invention is the concentration calculation system of an optically active substance according to the second aspect, wherein the calculating calculates values allocated to the unknown quantities related to the aqueous humor and the cornea such that the objective function becomes smaller than the case where other values are allocated for the objective function by varying the values in a stepwise manner as the concentration of the specific optically active substance.

A fourth aspect of the invention is a concentration calculation system of an optically active substance, comprising: a calculation unit configured to: acquire an amount of change in a polarization state by allowing light having different wavelengths to pass through a cornea and an aqueous humor; and calculate a concentration of a specific optically active substance contained in the aqueous humor by solving a simultaneous equation configured with equations of same number as the number of unknown quantities included in a theoretical formula based on the theoretical formula which includes a matrix representing a polarization property of the cornea and a matrix representing a polarization property of the aqueous humor and represents a wavelength dependence of the amount of change in the polarization state, wherein the matrix representing the polarization property of the aqueous humor is represented by a function of an expression representing the wavelength dependence of an optical rotation degree of the specific optically active substance and the expression includes a concentration value of the specific optically active substance as an unknown quantity.

A fifth aspect of the invention is a program causing a computer to execute a process for calculating a concentration of an optically active substance, the process comprising: acquiring an amount of change in a polarization state by allowing light having different wavelengths to pass through a cornea and an aqueous humor; and calculating a concentration of a specific optically active substance contained in the aqueous humor by a least squares method on the basis of a theoretical formula which includes a matrix representing a polarization property of the cornea and a matrix representing a polarization property of the aqueous humor and represents a wavelength dependence of the amount of change in the polarization state, wherein the matrix representing the polarization property of the aqueous humor is represented by a function of an expression representing the wavelength dependence of an optical rotation degree of the specific optically active substance and the expression includes a concentration value of the specific optically active substance as an unknown quantity or a temporal known quantity.

A sixth aspect of the invention is a program causing a computer to execute a process for calculating a concentration of a specific optically active substance contained in the aqueous humor by solving a simultaneous equation configured with equations of same number as the number of unknown quantities included in a theoretical formula based on the theoretical formula which includes a matrix representing a polarization property of the cornea and a matrix representing a polarization property of the aqueous humor and represents a wavelength dependence of the amount of change in the polarization state, wherein the matrix representing the polarization property of the aqueous humor is represented by a function of an expression representing the wavelength dependence of an optical rotation degree of the specific optically active substance and the expression includes a concentration value of the specific optically active substance as an unknown quantity.

According to the first aspect of the invention, it is possible to more accurately measure the concentration of the optically active substance contained in the aqueous humor compared to a case where influence by the cornea is not taken into account.

According to the second aspect of the invention, it is possible to prevent a concentration value of the optically active substance from becoming an irrational value compared to a case where the concentration of the optically active substances is calculated without using the first step and the second step.

According to the third aspect of the invention, it is possible to reduce an operation time required for calculation value of the concentration of the optically active substance compared to a case where the concentration is not varied in a stepwise manner.

According to the fourth to sixth aspects of the invention, it is possible to more accurately measure the concentration of the optically active substance contained in the aqueous humor compared to a case where influence by the cornea is not taken into account.

According to the seventh and eighth aspects of the invention, it is possible to more accurately measure the concentration of the optically active substance contained in the aqueous humor compared to a case where influence by the cornea is not taken into account.

BRIEF DESCRIPTION OF THE DRAWINGS

Fig. 1A and Fig. 1B are views illustrating an eyeball and an optical path, Fig. 1A is a sectional view taken along a plane formed by connecting from an internal canthus side to an outer canthus side of the eyeball, and Fig. 1B is a front view of the eyeball;

Fig. 2 is a diagram illustrating an outline of a concentration calculation system of an optically active substance according to a first exemplary embodiment;

Fig. 3 is a diagram illustrating functionality of an electrical signal processing unit;

Fig. 4 is a diagram illustrating a configuration of a concentration calculation unit;

Fig. 5 is a graph illustrating a relationship between an amount of change in a normalized polarization state and a wavelength $\lambda$;

Fig. 6 illustrates a Mueller matrix and a Stokes vector with respect to an optical system of the concentration calculation system of the optically active substance, a cornea of an eyeball, and aqueous humor of an anterior eye chamber that are objects to be measured illustrated in Fig. 2;

Fig. 7 is a table illustrating results obtained in Example 1;

Fig. 8 is a table illustrating results obtained using other initial values in Example 1;

Fig. 9 is a flowchart for obtaining the result in Example 2;

Fig. 10A and Fig. 10B are tables for illustrating results obtained by calculating a glucose concentration in a two-step method, Fig. 10A illustrates a first step that calculates an adjusted initial value, and Fig. 10B illustrates a second step that calculates the glucose concentration using the adjusted initial value;

Fig. 11 is a diagram illustrating an outline of a concentration calculation system of an optically active substance according to a second exemplary embodiment; and

Fig. 12 is a diagram illustrating an outline of a concentration calculation system of an optically active substance according to a third exemplary embodiment.

DETAILED DESCRIPTION

Hereinafter, an exemplary embodiment of the present invention will be described with reference to the accompanying drawings. First Exemplary Embodiment

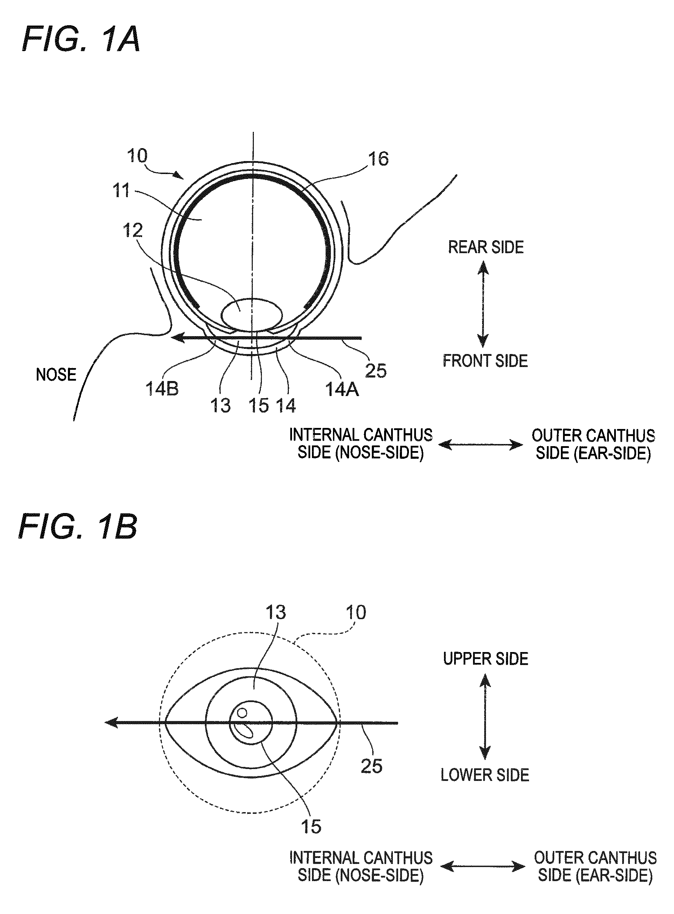

In the first exemplary embodiment, a concentration calculation system 1 of the optically active substance is illustrated in Fig. 1 which will be described later and calculates a concentration of the optically active substance such as glucose contained in aqueous humor of an anterior eye chamber 13 in the eyeball 10 of the human eye on the basis of a change in a polarization state of polarized light that passes through the anterior eye chamber 13.

(Eyeball 10 and Optical path 25)

Fig. 1A and Fig. 1B are views illustrating an eyeball 10 and an optical path 25. Fig. 1A is a sectional view taken along a plane formed by connecting from an internal canthus side to an outer canthus side of the eyeball 10, and Fig. 1B is a front view of the eyeball 10.

As illustrate in Fig. 1A, the eyeball 10 has an appearance that is a substantially spherical form and a glass body 11 is provided in the center of the eyeball 10. A crystalline lens 12 that functions as a lens is embedded in a portion of the glass body 11. The anterior eye chamber 13 is provided outside of the crystalline lens 12 and a cornea 14 is provided outside of the anterior eye chamber 13. A peripheral portion of the crystalline lens 12 is surrounded by an iris and a pupil 15 is provided in the center of the crystalline lens 12. The glass body 11 is covered with a retina 16 except for a portion that is in contact with the crystalline lens 12.

That is, the anterior eye chamber 13 is a region surrounded by the cornea 14 and the crystalline lens 12 and is protruded upward to be formed in a protruded shape from the sphere of the eyeball 10. The anterior eye chamber 13 is filled with aqueous humor.

As an example, the optical path 25 is set to be directed toward an internal canthus side (a nose-side) from an outer canthus side (an ear-side) and is provided to pass through an incidence side cornea 14A, an anterior eye chamber 13, and an emission side cornea 14B in this order.

The incidence side cornea 14A corresponds to a portion of the cornea 14 of a side onto which light is incident and the emission side cornea 14B corresponds to a portion of the cornea 14 of a side from which light is emitted.

As illustrate in Fig. 1B, in the front view of the eyeball 10, the optical path 25 is set to be directed toward the internal canthus side (nose-side) from the outer canthus side (ear-side).

Light advancing along the optical path 25 forms a beam shape.

Next, description will be made on the background of a calculation of a concentration of an optically active substance such as glucose from aqueous humor that is contained in the anterior eye chamber 13 of the eyeball 10.

An insulin dosage for a diabetic patient is controlled depending on a blood glucose concentration. The diabetic patient always needs to grasp a glucose concentration in the blood. The glucose concentration in the blood is called a blood sugar level and is widely used as an index of diabetes or the like. Measurement of the glucose concentration in the blood is usually performed by puncturing a fingertip or the like with an injection needle to collect a fine amount of blood. However, pain is accompanied with insertion of the needle into the fingertip when collecting a fine amount of blood. The demand for a non-invasive examining method instead of an invasive examining method such as puncturing is increasing.

In the eyeball 10, components of the aqueous humor of the anterior eye chamber 13 are almost the same as serum and contain protein, glucose, ascorbic acid and the like. It is known that there is a correlation between a glucose concentration in the blood and a glucose concentration in the aqueous humor. Furthermore, intra-cellular substances are not present in the aqueous humor and are less affected by light scattering. The protein, the glucose, ascorbic acid and the like contained in the aqueous humor are the optically active substances and have optical activity. The aqueous humor is advantageous as a portion for optically measuring a concentration of glucose or the like using the optical activity. Performing the optical measurement of a concentration of glucose or the like may result in a non-invasive examining method.

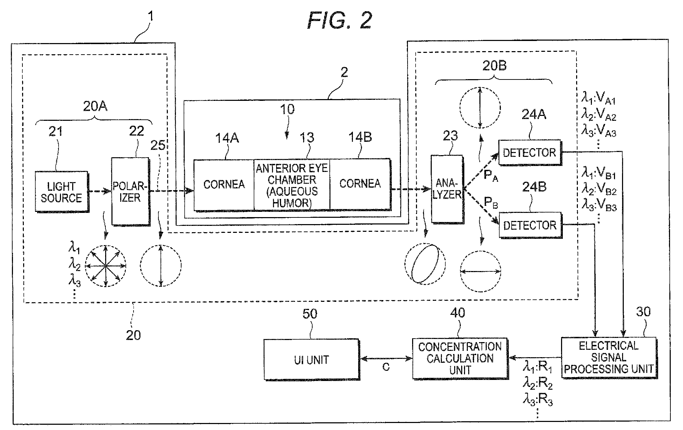

In the method for optically obtaining the concentration of the optically active substance contained in the aqueous humor, an optical path to which light is incident at an angle substantially perpendicular to the eyeball 10 may be considered as an optical path capable of being set other than the optical path 25 which cuts across the anterior eye chamber 13 of the eyeball 10 described above. In the optical path to which light is incident at an angle substantially perpendicular to the eyeball 10, light is reflected from an interface between the aqueous humor and the iris or an interface between the aqueous humor or the crystalline lens 12 and receives light reflecting from the interface in the anterior eye chamber 13. In the optical path, light is likely to reach the retina 16. In particular, in a case where a laser having high coherence is used in the light source 21 (see Fig. 2 which will be described later), it is not preferable that light reaches the retina 16.

The concentration calculation system 1 of the optically active substance which will be described later may also be applied to the optical path to which light is incident at an angle substantially perpendicular to the eyeball 10.

Even in a case where the optical path 25 is set to cut across the anterior eye chamber 13 of the eyeball 10, the optical path 25 set to be directed toward the internal canthus side (nose-side) from the outer canthus side (ear-side), however, the optical path 25 may be set to be directed toward the outer canthus side (ear-side) from the internal canthus side (nose-side)in Fig. 1A and Fig. 1B. The optical path 25 may be set to be directed toward a lower side from an upper side or toward the upper side from the lower side, or may be set to be directed toward an oblique lower side direction from an oblique upper side direction or toward the oblique upper side direction from the oblique lower side direction.

(Concentration calculation system 1 of the optically active substance)

Fig. 2 is a diagram illustrating an outline of a concentration calculation system 1 of an optically active substance according to the first exemplary embodiment.

The optically active substance has optical activity which rotates a polarization plane of the linearly polarized light which is irradiated. In the present specification, the polarization plane refers to a plane, in which the electric field vibrates, of the linearly polarized light.

The concentration calculation system 1 of the optically active substance illustrated in Fig. 2 irradiates the linearly polarized light to the anterior eye chamber 13 of the eyeball 10 containing the optically active substance through the cornea 14 (incidence side cornea 14A and emission side cornea 14B) and calculates a concentration of the optically active substance contained in the aqueous humor of the anterior eye chamber 13 which is an object to be measured 2 from an amount of change in the polarization state observed after passing through the object to be measured 2.

Plural optically active substances are contained in the aqueous humor of the anterior eye chamber 13, but it may be sufficient to determine a concentration of an optically active substance intended to be obtained, for example, glucose. In a case where the plural optically active substances are contained in the aqueous humor of the anterior eye chamber 13, the concentration of the optically active substance intended to be obtained is a concentration of an optically active substance that a user wants to know and is a concentration of the optically active substance that becomes a target to be displayed.

The concentration calculation system 1 of the optically active substance includes an optical system 20, an electrical signal processing unit 30, a concentration calculation unit 40, and a user interface (UI) unit 50. The optical system 20 is connected to the electrical signal processing unit 30, the electrical signal processing unit 30 is connected to the concentration calculation unit 40, and the concentration calculation unit 40 is connected to the UI unit 50. The connections may be made wirelessly or through a wired line.

The optical system 20 includes a light source unit 20A which allows the linearly polarized light to be incident on the object to be measured 2 (here, anterior eye chamber 13 of the eyeball 10) and a light receiving unit 20B which receives light passing through the object to be measured 2 to be emitted. A light source 21 of the light source unit 20A, which will be described later, emits light having plural wavelengths $\lambda_1, \lambda_2, \lambda_3, ....$ The light receiving unit 20B outputs voltages $V_{A1}, V_{A2}, V_{A3}, ...$ and voltages $V_{B1}, V_{B2}, V_{B3}, ...,$ that are electric signals corresponding to light intensities for the plural wavelengths $\lambda_1, \lambda_2, \lambda_3, ...,$ to the electrical signal processing unit 30.

The electrical signal processing unit 30 outputs an amount of change in polarization states $R_1, R_2, R_3, ...,$ that are normalized for the plural wavelengths $\lambda_1, \lambda_2, \lambda_3, ...$ from the voltages $V_{A1}, V_{A2}, V_{A3}, ...$ and voltages $V_{B1}, V_{B2}, V_{B3}, ...$ corresponding to intensities of light output from the light receiving unit 20B of the optical system 20, to the concentration calculation unit 40. In the following, the voltages $V_{A1}, V_{A2}, V_{A3}, ...$ and the voltages $V_{B1}, V_{B2}, V_{B3}, ...$ that represent the light intensities are denoted by light intensity voltages $V_{A1}, V_{A2}, V_{A3}, ...$ and light intensity voltages $V_{B1}, V_{B2}, V_{B3}, ....$

The concentration calculation unit 40 calculates a concentration C of a specific optically active substance contained in the aqueous humor of the anterior eye chamber 13 from the amount of change in polarization states $R_1, R_2, R_3, ...,$ that are normalized for the plural wavelengths $\lambda_1, \lambda_2, X_3, ...,$ output from the electrical signal processing unit 30 by numerical calculation processing and outputs the concentration C to the UI unit 50.

The concentration C of the optically active substance may be denoted by a concentration value.

The UI unit 50 includes an input device, such as a key keyboard, for inputting instructions or data by the user and an output device, such as a display, for displaying processing results to the user.

The user inputs an expression or an eigenvalue which will be described later to the concentration calculation unit 40 through the input device, such as the keyboard, of the UI unit 50.

The user obtains the concentration C of the optically active substance intended to be obtained, which is calculated in the concentration calculation unit 40, through the output device, such as the display, of the UI unit 50.

In a case where the wavelengths $\lambda_1, \lambda_2, \lambda_3, ...,$ the light intensity voltages $V_{A1}, V_{A2}, V_{A3}, ...,$ the light intensity voltages $V_{B1}, V_{B2}, V_{B3}, ...,$ and the amount of change in the normalized polarization states $R_1, R_2, R_3, ...$ are not respectively distinguished from each other or in a case where description is made on a single wavelength which is adopted as a representative of the wavelengths, it is denoted as a wavelength $\lambda$, a light intensity voltage $V_A$, a light intensity voltage $V_B$, and an amount of change in the normalized polarization state R.

Next, details of the optical system 20 will be described.

The optical system 20 includes a light source 21 that emits light of a predetermined wavelength $\lambda$, a polarizer 22 that extracts linearly polarized light having a predetermined polarization plane from the light emitted by the light source 21, an analyzer 23 which allows the linearly polarized light having the predetermined polarization plane to pass therethrough, and a detector 24A and a detector 24B that receive light passing through the analyzer 23. In a case where the detector 24A and the detector 24B are not distinguished from each other, it is denoted by a detector 24.

In the optical system 20 illustrated in Fig. 2, shapes of polarization images, when viewed from a propagation direction of light, are represented by encircled arrows between the light source 21, the polarizer 22, the eyeball 10, the analyzer 23, and the detectors 24A and 24B.

The light source 21 may be a light emitting diode (LED), a light source such as a lamp having a wide wavelength (spectrum) width, or a light source such as a laser having a narrow wavelength (spectrum) width. The light source such as a laser having a narrow wavelength (spectrum) width is preferable.

A light source that emits light having at least two or more wavelengths $\lambda$ is used for the light source 21. Furthermore, as the light source 21, for example, a light source having the wavelength range included in a region, in which optical rotation degree $\phi_{AH}$ of the optically active substance contained in the aqueous humor of the anterior eye chamber 13 is able to be approximated by the Drude monomial, which will be described later, is used. The wavelength range is, for example, 400 nm to 900 nm.

Here, light emitted from the light source 21 contains light having a random polarization plane as illustrated in Fig. 2. The light source 21 may be a light source that emits linearly polarized light. In such a case, the polarizer 22 which will be described later may not be utilized.

The polarizer 22 is, for example, a total reflection type Glan-Thompson prism, a total reflection type Glan-Taylor prism, and a total reflection type Glan-Laser prism, and allows linearly polarized light having the predetermined polarization plane from light having a random polarization plane and emitted from the light source 21 to pass through. In Fig. 2, for example, the polarization plane the linearly polarized light having the polarization plane that is parallel to a paper plane is allowed to pass through.

When the linearly polarized light, that passes through the polarizer 22, passes through the incidence side cornea 14A, the aqueous humor of the anterior eye chamber 13, and the emission side cornea 14B in the eyeball 10, the linearly polarized light becomes elliptically polarized light.

The incidence side cornea 14A and the emission side cornea 14B have birefringence and the aqueous humor in the anterior eye chamber 13 has the optical activity. For that reason, the input linearly polarized light becomes elliptically polarized light and is emitted.

The analyzer 23 is for example, a polarization separation type Wollaston prism and Rochon prism, and separates incident light into two linearly polarized lights orthogonal to each other and emits the linearly polarized light. Light intensity of one linearly polarized light emitted from the analyzer 23 is regarded as the light intensity $P_A$ and the light intensity of the other linearly polarized light is regarded as the light intensity $P_B$.

Here, in a case where the object to be measured 2 is not present, that is, in a case where light emitted from the light source 21 passes through the polarizer 22 and then, is directly incident onto the analyzer 23, the analyzer 23 is arranged in such a way that the light intensities $P_A$ and $P_B$ of the two separated linearly polarized lights that are orthogonal to each other become identical to each other.

The detector 24A and the detector 24B are photo-detectors such as silicon diodes and output electric signals corresponding to the light intensity $P_A$ and the light intensity $P_B$ of the incident light.

The detector 24A to which one linearly polarized light (light intensity $P_A$) of the linearly polarized light emitted from the analyzer 23 is incident and the detector 24A outputs the light intensity voltage $V_A$ corresponding to the light intensity $P_A$. The detector 24B to which the other linearly polarized light (light intensity $P_B$) of the linearly polarized light emitted from the analyzer 23 is incident and the detector 24B outputs the light intensity voltage $V_A$ corresponding to the light intensity $P_B$.

In a case where the object to be measured 2 is not present, the light intensities $P_A$ and $P_B$ of the two separated linearly polarized lights that are emitted by the analyzer 23 and orthogonal to each other become identical to each other and thus, the light intensity voltages $V_A$ and $V_B$ become identical to each other.

The optical system 20 as described above is an example and may have a configuration including other optical elements such as a mirror, a lens, a wavelength plate, a prism or the like.

(Electrical Signal Processing Unit 30)

Next, the electrical signal processing unit 30 will be described.

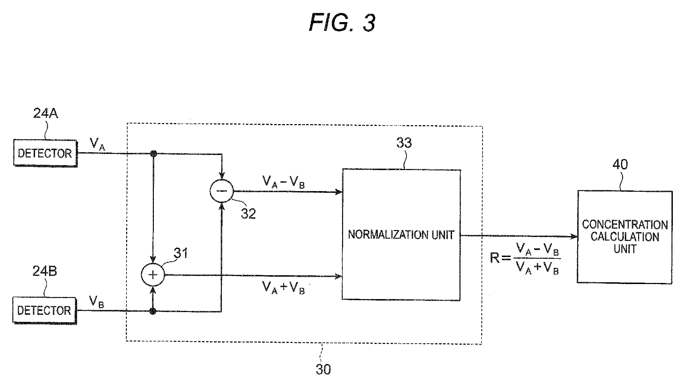

Fig. 3 is a diagram illustrating functionality of an electrical signal processing unit 30.

The electrical signal processing unit 30 includes an addition unit 31 (denoted by a symbol of encircled "+" in Fig. 3), a subtraction unit 32 (denoted by a symbol of encircled "-" in Fig. 3), and a normalization unit 33. Here, description will be made on a single wavelength $\lambda$. Accordingly, the light intensity voltage $V_A$ and the light intensity voltage $V_B$ are used as light intensity voltages.

The light intensity voltage $V_A$ from the detector 24A and the light intensity voltage $V_B$ from the detector 24B are input to the addition unit 31 and the subtraction unit 32. The addition unit 31 outputs a voltage ($V_A + V_B$) of a sum of the light intensity voltage $V_A$ and the light intensity voltage $V_B$ to the normalization unit 33. The subtraction unit 32 outputs a voltage ($V_A - V_B$) of a difference of the light intensity voltage $V_A$ and the light intensity voltage $V_B$ to the normalization unit 33.

The normalization unit 33 computes an amount of change in the normalized polarization state R from the voltage ($V_A + V_B$) of the sum output from the addition unit 31 and the voltage ($V_A - V_B$) of the difference output from the subtraction unit 32 according to Equation (1). The normalization unit 33 outputs the amount of change in the normalized polarization state R to the concentration calculation unit 40.

$$R = \frac{V_A - V_B}{V_A + V_B} \qquad \cdots (1)$$

In a case where there is no loss, the voltage ($V_A + V_B$) of the sum of the light intensity voltage $V_A$ and the light intensity voltage $V_B$ corresponds to the light intensity before light is incident to the analyzer 23. In a case where the object to be measured 2 is not present, the voltage ($V_A - V_B$) of the difference between the light intensity voltage $V_A$ and the light intensity voltage $V_B$ becomes "0". Accordingly, the voltage ($V_A - V_B$) of the difference corresponds to the amount of change in the polarization state that is generated due to the presence of the object to be measured 2. The R is an amount of change in the normalized polarization state.

The electrical signal processing unit 30 is configured with hardware such as an analog electronic circuit and may allow the amount of change in the normalized polarization state R to be output when the light intensity voltage $V_A$ is input from the detector 24A and the light intensity voltage $V_B$ is input from the detector 24B.

The electrical signal processing unit 30 is configured as a computer provided with a CPU, a memory or the like, and may calculate the amount of change in the normalized polarization state R by operation processing from the input light intensity voltage $V_A$ and the input light intensity voltage $V_B$ by software, similar to the concentration calculation unit 40 which will be described later.

(Concentration Calculation Unit 40)

Next, descriptions will be made on a concentration calculation method performed by the concentration calculation unit 40.

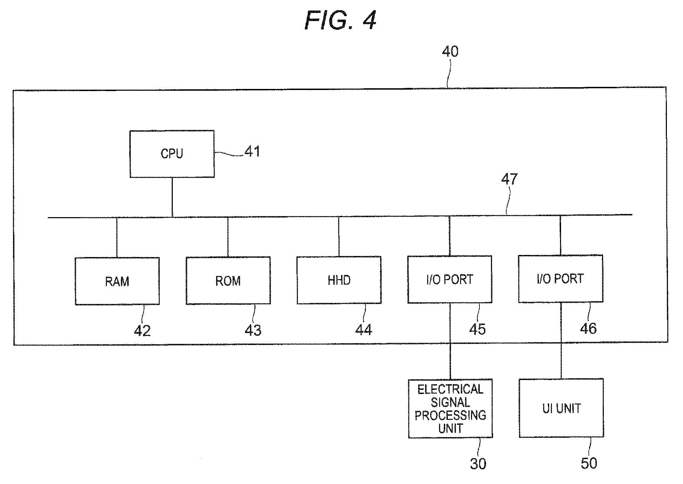

Fig. 4 is a diagram illustrating a configuration of the concentration calculation unit 40. The concentration calculation unit 40 includes an operation processing unit (denoted by a CPU in Fig. 4) 41, a random access memories (RAM) 42, a read only memory (ROM) 43, a hard disk drive (HDD) 44, and an input/output port (I/O port) 45 and an I/O port 46. The CPU 41, the RAM 42, the ROM 43, the HDD 44, and the I/O port 45 and the I/O port 46 are connected to a bus 47.

The I/O port 45 is connected with the electrical signal processing unit 30 and the I/O port 46 is connected with the UI unit 50.

That is, the concentration calculation unit 40 is configured as a computer.

The CPU 41 deploys a program (software) or data for calculating the concentration C of the optically active substance stored in the ROM 43 or the HDD 44 on the RAM 42 and executes the program to calculate the concentration C of the optically active substance.

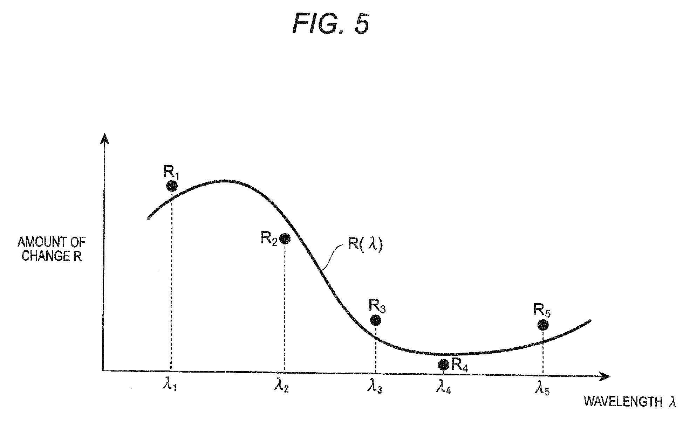

Fig. 5 is a graph illustrating a relationship between an amount of change in a normalized polarization state R and the wavelength λ. In Fig. 5, the amount of change in the normalized polarization state R is denoted by an amount of change R.

As described in Fig. 2, the amount of change in the normalized polarization states $R_1$, $R_2$, $R_3$, ..., that respectively correspond to the plural wavelengths $\lambda_1$, $\lambda_2$, $\lambda_3$, ... are input to the concentration calculation unit 40 from the electrical signal processing unit 30.

The concentration calculation unit 40 uses a function (theoretical value) R(λ) which is a function of the wavelength λ. In the nonlinear least squares method, a sum of squares of a difference ($R(\lambda_i) - R_i$) between the amount of change in the normalized polarization state Ri that is input and a function R ($\lambda_i$) is used as an objective function and calculates a value of an unknown quantity of the function R(λ) in a case where the objective function becomes smaller (to the minimum) than a case where other values are used for the objective_function. The "i" is an integer of one or more.

The concentration C of the optically active substance is included in the unknown quantities of the function R(λ), which will be described later, and thus, the concentration is calculated.

The objective function is represented by Equation (2).

$$\sum_{i=1}^{\substack{\text{Wavelength} \\ \text{number}}} \left[R(\lambda_i) - R_i\right]^2 \qquad \cdots (2)$$

In the nonlinear least squares method, the unknown quantity (variable) may be applied to the function $R(\lambda)$ while varying (changing) the unknown quantity such that a sum of squares of a difference between the function $R(\lambda)$ and the amount of change in the normalized polarization state R, which is a measured value, becomes the minimum. In the nonlinear least squares method, an algorithm such as the Levenberg-Marquardt method, the quasi-Newton method, the conjugate gradient method, or the like is used.

Here, a concentration C of the optically active substance intended to be obtained is calculated using the Levenberg-Marquardt method. The Levenberg-Marquardt method is developed to improve instability in convergence of a solution in the Gauss-Newton method and is widely used as the algorithm of the nonlinear least squares method. The Levenberg-Marquardt method is a well-known method and thus description thereof will be omitted.

As the measured value, the plural amounts of change in the normalized polarization state R, that is, amounts of change in the normalized polarization states R for two or more wavelengths $\lambda$ are used. Accuracy of the unknown quantity intended to be obtained is gradually improved as the number of wavelengths $\lambda$ is increased. However, when the number of wavelengths $\lambda$ is increased, it takes time to perform the numerical calculation. The number of wavelengths $\lambda$ may be selected in accordance with a situation.

Although the Levenberg-Marquardt method is used, other algorithms may also be applied.

The plural functions $R(\lambda)$ regarding the plural wavelengths $\lambda$ may be simultaneously set up to work out the simultaneous equations to thereby calculate the concentration C of the optically active substance intended to be obtained, instead of calculating the concentration C of the optically active substance intended to be obtained in the nonlinear least squares method described above. In a case of solving the simultaneous linear equations, the number of wavelengths $\lambda$ for measurement and the number of unknown quantities are required to be equal to each other.

On the other hand, applying the function by the nonlinear least squares method using the Levenberg-Marquardt method or the like, the concentration C of the optically active substance intended to be obtained is able to be calculated even when the number of wavelengths $\lambda$ is not equal to the number of unknown quantities. That is, when the nonlinear least squares method is adopted, any number of wavelengths $\lambda$ may be used for measurement regardless of whether the number of wavelengths $\lambda$ is greater than or less than the number of unknown quantities.

In the following, the nonlinear least squares method is denoted by the least squares method.

(Function $R(\lambda)$)

A function $R(\lambda)$ will be described.

In the concentration calculation system 1 of the optically active substance, the polarization properties of the optical system 20, and the cornea 14 of the eyeball 10 and the aqueous humor of the anterior eye chamber 13, that are objects to be measured 2, are described by a product of the Mueller matrices M which represent respective polarization properties. In the following, although a suffix is attached to distinguish the Mueller matrices M representing the respective polarization properties, it is denoted by the Mueller matrix M in a case of not distinguishing the Mueller matrices M.

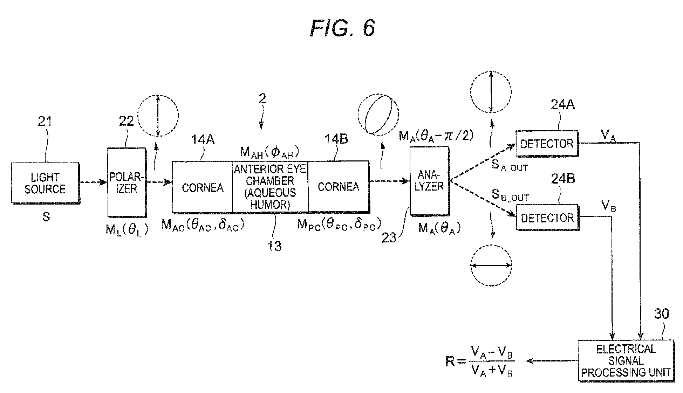

Fig. 6 illustrates a Mueller matrix M and a Stokes vector S with respect to the optical system 20 of the concentration calculation system 1 of the optically active substance, the cornea 14 of an eyeball 10, and the aqueous humor of the anterior eye chamber 13 that are objects to be measured 2 illustrated in Fig. 2.

A Mueller matrix $M_L$ ($\theta_L$) (L: Linear Polarizer) of the polarizer 22 is represented by a function of a rotation angle $\theta_L$, which will be described later.

The cornea 14 has birefringence and thus the Mueller matrix $M_{AC}$ ($\theta_{AC}$, $\delta_{AC}$) (AC: Anterior cornea) of the incidence side cornea 14A is represented by a function of an inclination $\theta_{AC}$ of the fast axis and a phase difference $\delta_{AC}$ between the fast axis and the slow axis.

The aqueous humor has optical activity and thus the Mueller matrix $M_{AH}$ ($\phi_{AH}$) (AH: Aqueous Humor) of the aqueous humor of the anterior eye chamber 13 is represented by a function of the optical rotation degree $\phi_{AH}$.

The Mueller matrix $M_{PC}$ ($\theta_{PC}$, $\delta_{PC}$) (PC: Posterior Cornea) of the emission side cornea 14B is represented by a function of an inclination $\theta_{PC}$ of the fast axis and a phase difference $\delta_{PC}$ between the fast axis and the slow axis, similar to the incidence side cornea 14A.

The Mueller matrix $M_A$ ($\theta_A$) (A: Analyzer) of the analyzer 23 is represented by a function of a rotation angle $\theta_A$, similar to the Mueller matrix $M_L$ ($\theta_L$) of the polarizer 22.

The Mueller matrix M of the detector 24A side is represented by Equation (3) and the Mueller matrix M of the detector 24B side is represented by Equation (4).

$$M_A\left(\theta_A - \frac{\pi}{2}\right) \cdot M_{PC}(\theta_{PC}, \delta_{PC}) \cdot M_{AH}(\phi_{AH}) \cdot M_{AC}(\theta_{AC}, \delta_{AC}) \cdot M_L(\theta_L) \quad \cdots (3)$$

$$M_A(\theta_A) \cdot M_{PC}(\theta_{PC}, \delta_{PC}) \cdot M_{AH}(\phi_{AH}) \cdot M_{AC}(\theta_{AC}, \delta_{AC}) \cdot M_L(\theta_L) \quad \cdots (4)$$

Here, light emitted from the light source 21 is represented by the Stokes vector S. The Stokes vector S may be referred to as Stokes parameters.

One linearly polarized light component, which is incident on the detector 24A, of two orthogonal linear polarized lights separated by the analyzer 23 is represented by a Stokes vector $S_{A\_OUT}$ and the other linearly polarized light which is incident on the detector 24B is represented by the Stokes vector $S_{B\_OUT}$. Then, using Equation (3) and Equation (4), the Stokes vector $S_{A\_OUT}$ is represented by Equation (5) and the Stokes vector $S_{B\_OUT}$ is represented by Equation (6).

$$S_{A\_OUT} = M_A\left(\theta_A - \frac{\pi}{2}\right) \cdot M_{PC}(\theta_{PC}, \delta_{PC}) \cdot M_{AH}(\phi_{AH}) \cdot M_{AC}(\theta_{AC}, \delta_{AC}) \cdot M_L(\theta_L) \cdot S \quad \cdots (5)$$

$$S_{B\_OUT} = M_A(\theta_A) \cdot M_{PC}(\theta_{PC}, \delta_{PC}) \cdot M_{AH}(\phi_{AH}) \cdot M_{AC}(\theta_{AC}, \delta_{AC}) \cdot M_L(\theta_L) \cdot S \quad \cdots (6)$$

The Stokes vector S is a matrix consisting of four rows by a single column and having four quantities $S_0$, $S_1$, $S_2$, and $S_3$ as elements, all of which have a strength dimension, and is represented by Equation (7).

$$S = \begin{pmatrix} S_0 \\ S_1 \\ S_2 \\ S_3 \end{pmatrix} \quad \cdots (7)$$

Here, an So component of the Stokes vector S corresponds to the light intensity. The light intensity voltage $V_A$ and light intensity voltage $V_B$ of light that passes through the analyzer 23 correspond to So components of the Stokes vector $S_{A\_OUT}$ and the Stokes vector $S_{B\_OUT}$, The light intensity voltages $V_A$ and $V_B$ are represented by Equation (8) and Equation (9).

The amount of change in the normalized polarization state R is represented by Equation (10).

$$V_A = \left(S_{A\_OUT}\right)_0 \quad \cdots (8)$$

$$V_B = \left(S_{B\_OUT}\right)_0 \quad \cdots (9)$$

$$R = \frac{V_A - V_B}{V_A + V_B} = \frac{(S_{A\_OUT})_0 - (S_{B\_OUT})_0}{(S_{A\_OUT})_0 + (S_{B\_OUT})_0}$$
$$= \frac{\left(\left[M_A\left(\theta_A - \frac{\pi}{2}\right) - M_A(\theta_A)\right] \cdot M_{PC}(\theta_{PC}, \delta_{PC}) \cdot M_{AH}(\phi_{AH}) \cdot M_{AC}(\theta_{AC}, \delta_{AC}) \cdot M_L(\theta_L) \cdot S\right)_0}{\left(\left[M_A\left(\theta_A - \frac{\pi}{2}\right) + M_A(\theta_A)\right] \cdot M_{PC}(\theta_{PC}, \delta_{PC}) \cdot M_{AH}(\phi_{AH}) \cdot M_{AC}(\theta_{AC}, \delta_{AC}) \cdot M_L(\theta_L) \cdot S\right)_0} \quad \cdots (10)$$

Descriptions will be made on the Mueller matrix $M_L$ ($\theta_L$) of the polarizer 22, the Mueller matrix $M_{AC}$ ($\theta_{AC}$, $\delta_{AC}$) of the incidence side cornea 14A, and the Mueller matrix $M_{AH}$ ($\phi_{AH}$) of the aqueous humor of the anterior eye chamber 13.

The Mueller matrix $M_L$ ($\theta_L$) of the polarizer 22 is represented by Equation (11). The Mueller matrix $M_A$ ($\theta_A$) of the analyzer 23 is obtained by replacing "L" with "A" in the Mueller matrix $M_L$ ($\theta_L$) of the polarizer 22. Descriptions on the Mueller matrix $M_A$ ($\theta_A$) of the analyzer 23 will be omitted.

The "A" in the Mueller matrix $M_{AC}$ ($\theta_{AC}$, $\delta_{AC}$) of the incidence side cornea 14A is replaced with "P" in the Mueller matrix $M_{PC}$ ($\theta_{PC}$, $\delta_{PC}$) of the emission side cornea 14B. Here, a Mueller matrix MC for the cornea 14 is denoted by the Mueller matrix $M_C$ ($\theta_C$, $\delta_C$) without distinguishing the incidence side cornea 14A and the emission side cornea 14B. The Mueller matrix $M_C$($\theta_C$, $\delta_C$) of the cornea 14 is represented by Equation (12).

The Mueller matrix $M_{AH}$ ($\phi_{AH}$) of the aqueous humor of the anterior eye chamber 13 is represented by Equation (13).

$$M_L(\theta_L) = \frac{1}{2} \cdot \begin{pmatrix} 1 & \cos2\theta_L & \sin2\theta_L & 0 \\ \cos2\theta_L & \cos^2 2\theta_L & \cos2\theta_L \cdot \sin2\theta_L & 0 \\ \sin2\theta_L & \cos2\theta_L \cdot \sin2\theta_L & \sin^2 2\theta_L & 0 \\ 0 & 0 & 0 & 0 \end{pmatrix} \qquad \cdots (11)$$

$$M_C(\theta_C, \delta_C) =$$
$$\begin{pmatrix} 1 & 0 & 0 & 0 \\ 0 & \cos^2 2\theta_C + \cos\delta_C \cdot \sin^2 2\theta_C & \cos2\theta_C \cdot \sin2\theta_C - \cos2\theta_C \cdot \cos\delta_C \cdot \sin2\theta_C & -\sin2\theta_C \cdot \sin\delta_C \\ 0 & \cos2\theta_C \cdot \sin2\theta_C - \cos2\theta_C \cdot \cos\delta_C \cdot \sin2\theta_C & \cos\delta_C \cdot \cos^2 2\theta_C + \sin^2 2\theta_C & \cos2\theta_C \cdot \sin\delta_C \\ 0 & \sin2\theta_C \cdot \sin\delta_C & -\cos2\theta_C \cdot \sin\delta_C & \cos\delta_C \end{pmatrix} \quad \cdots (12)$$

$$M_{AH}(\phi_{AH}) = \begin{pmatrix} 1 & 0 & 0 & 0 \\ 0 & \cos2\phi_{AH} & \sin2\phi_{AH} & 0 \\ 0 & -\sin2\phi_{AH} & \cos2\phi_{AH} & 0 \\ 0 & 0 & 0 & 1 \end{pmatrix} \qquad \cdots (13)$$

In the object to be measured 2 containing any single optically active substance, optical rotation degree $\phi$ for the wavelength $\lambda$ is represented by a product of an optical path length L and a concentration C. Here, the optically active substance in the anterior eye chamber 13 is not necessarily the aqueous humor and thus, it is denoted by the optical rotation degree $\phi$.

The optical rotation degree $\phi$ is represented by the Drude monomial which is a nonlinear function that monotonously decreases or monotonously increases in a wavelength of which the length is longer than the maximum point and the minimum point. The Drude monomial is an example of a function which represents a distribution of optical rotations of the optically active substances.

In a case where the object to be measured contains plural optically active substances, the optical rotation degree $\phi$ to be observed is expressed by adding an optical rotation degree $\phi_j$ of each optically active substance represented by the Drude monomial. In other words, the optical rotation degree $\phi$ to be observed of each optically active substance is represented by a sum of functions, each of which represents wavelength dependence of the optical rotation degree $\phi_j$ of each optically active substance. The "j" is an integer of one or more.

As an example, the optical rotation degree $\phi_{AH}$ due to the aqueous humor of the anterior eye chamber 13 is represented by the sum of two Drude monomials expressed in Equation (14). In a case where glucose is regarded as the optically active substance intended to be obtained, a first term of the right side is a term subjected to contribution by glucose. A second term of the right side is a term subjected to contribution by other optically active substances except for glucose.

The concentration of glucose is set as a glucose concentration Cg. The Ag and $\lambda_g$ are unique constants (eigenvalue regulating optical rotation distribution of optically active substances (glucose)) unique to the optically active substance (glucose).

Furthermore, the $A_x$ and $\lambda_x$ are eigenvalues in a case where other optically active substances are collected.

The "L" is a length of the optical path.

The optical rotation degree $\phi_{AH}$ due to the aqueous humor of the anterior eye chamber 13 is represented by a function of the $A_x$ and the $\lambda_x$ that are eigenvalues in a case where the glucose concentration Cg and other optically active substances are collected.

$$\phi_{AH}\left(C_g, A_x, \lambda_x\right) = L \cdot \left( \frac{A_g}{\lambda^2 - \lambda_g^2} \cdot C_g + \frac{A_x}{\lambda^2 - \lambda_x^2} \right) \qquad \cdots (14)$$

In Equation (14), other optically active substances, that are contained in the aqueous humor of the anterior eye chamber except for glucose, are collected, but the optical rotation degree $\phi_{AH}$ may be represented by plural terms for other optically active substances. For example, in addition to a term for glucose, a term for albumin, a term for globulin or the like may be provided. In this case, terms for other optically active substances, except for the optically active substances (glucose, albumin, globulin or the like) for which the terms are provided, may be set.

Furthermore, in a case where contributions by other optically active substances are small or the like, the terms for other optically active substances may not be provided.

That is, a term may be set in consideration of a degree of influence to optical rotation degree $\phi_{AH}$ due to the optically active substance intended to be obtained and the aqueous humor of the anterior eye chamber 13.

The phase difference $\delta_C(\lambda)$ in the cornea 14 is represented by Equation (15). The phase difference $\delta_C(\lambda)$ is represented by a thickness d of the cornea 14 and a difference in refractive indices $\Delta n$ between the fast axis and the slow axis.

$$\delta_C(\lambda) = \frac{2\pi}{\lambda} \cdot d \cdot \Delta n \qquad \cdots (15)$$

As described above, the amount of change in the normalized polarization state R is represented by a product of the Mueller matrix M which represents polarization properties of the optical system 20, the cornea 14 (incidence side cornea 14A and emission side cornea 14B) of the eyeball 10, and the aqueous humor of the anterior eye chamber 13 and the Stokes vector S of the light source 21.

When the plural wavelengths $\lambda$ and the amounts of changes in the normalized polarization states R to the plural wavelengths $\lambda$ are input, the concentration calculation unit 40 applies the least squares method to Equation (10) into which Equation (14) and Equation (15) are substituted and calculates plural unknown quantities containing the glucose concentration Cg.

In the following, description will be specifically made on a method for calculating the concentration C of the optically active substance.

Here, it is regarded that the aqueous humor of the anterior eye chamber 13 contains only glucose, and Equation (16) is used instead of Equation (14).

$$\phi_{AH}(\lambda) = \frac{A_g}{\lambda^2 - \lambda_g^2} \cdot L \cdot C_g \qquad \cdots (16)$$

The eigenvalues of glucose Ag and $\lambda_g$ are respectively set to $1.72 \times 10^7$ and 150. The optical path length L in the aqueous humor of the anterior eye chamber 13 is set to 1 cm.

Furthermore, in the optical system 20, the rotation angle $\theta_A$ of the polarizer 22 is set to $\pi/4$ rad and the rotation angle $\theta_L$ of the analyzer 23 is set to 0 rad.

The inclination $\theta_{AC}$ of the fast axis of the incidence side cornea 14A, the inclination $\theta_{PC}$ of the fast axis of the emission side cornea 14B, the thickness d of the cornea 14, the difference in refractive indices $\Delta n$ between the fast axis and the slow axis of the cornea 14, and the glucose concentration Cg are respectively set to unknown quantities.

The thickness d and the refractive index difference $\Delta n$ of the cornea 14 consist of a thickness da and a refractive index difference $\Delta na$ with respect to the incidence side cornea 14A and a thickness dp and a refractive index difference $\Delta np$ with respect to the emission side cornea 14B. The product da·$\Delta na$ of the thickness and the refractive index and the product dp·$\Delta np$ of the thickness and the refractive index are respectively set to the unknown quantities.

The unknown quantities are five unknown quantities consisting of the inclination $\theta_{AC}$ of the fast axis of the incidence side cornea 14A, the inclination $\theta_{PC}$ of the fast axis of the emission side cornea 14B, the product da·$\Delta na$, the product dp·$\Delta np$, and the glucose concentration $C_g$. The five unknown quantities are obtained by simulation to which the least squares method is applied.

(Example 1)

In Example 1, the five unknown quantities are used as variables, the least squares method is repeated while varying the unknown quantity from an initial value, and a value (result) of a variable (unknown quantity), with which the objective function of Equation (2) becomes smaller (to the minimum) than a case where other values are used for the objective function, is calculated.

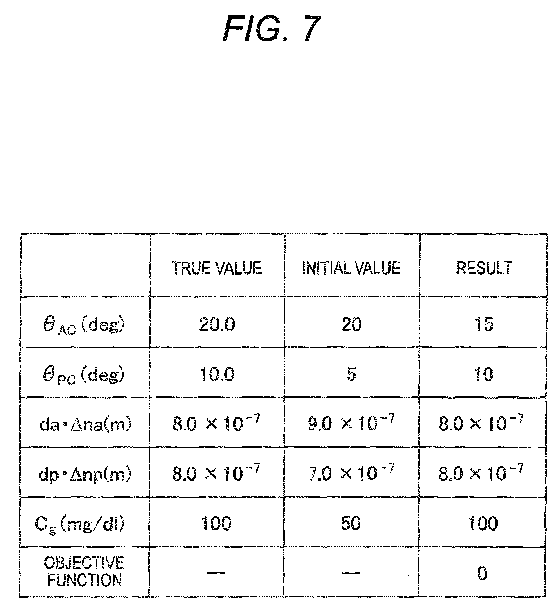

Fig. 7 is a table illustrating results obtained in Example 1.

The unknown quantities correspond to the five unknown quantities described above. Respective initial values (third column) are set with respect to true values (second column) that are set, and results (fourth column) are obtained.

The calculation is started by setting the initial value to 50 mg/dl with respect to the glucose concentration $C_g$ of which a true value is 100 mg/dl and 100 mg/dl, which is the same as the true value, is obtained as a result of the calculation. The inclination $\theta_{AC}$ of the fast axis of the incidence side cornea 14A, which is obtained as a result, is deviated from the true value, but coincides with the inclination $\theta_{PC}$ of the fast axis of other emission side cornea 14B, and also coincides with the true values of the product $da \cdot \Delta na$ and the product $dp \cdot \Delta np$.

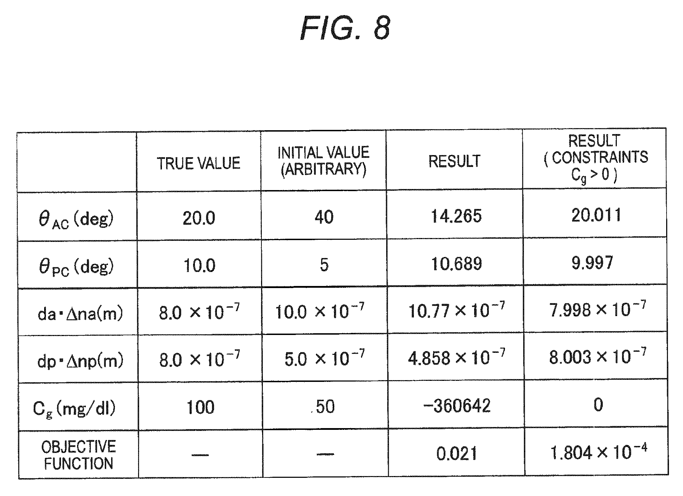

Fig. 8 is a table illustrating results obtained using other initial values in Example 1.

Here, initial values (third column) different from those of Fig. 7 are used. In the obtained results (fourth column), the glucose concentration $C_g$ becomes an irrational value as - 360642 mg/dl.

A constraint condition that the glucose concentration $C_g$ is a positive concentration value ($C_g>0$) is applied to the initial values (third column) and results (fifth column) are obtained again. However, the obtained glucose concentration $C_g$ is 0 mg/dl and the glucose concentration $C_g$ of 100 mg/dl which is a true value is not obtained.

That is, the obtained result becomes greatly different due to the initial value to be set.

When a change in the polarization state generated by the aqueous humor of the anterior eye chamber 13 is compared with a change in the polarization state by the cornea 14, the change in the polarization state by the cornea 14 is significantly greater than that of the aqueous humor.

For that reason, as in Example (1), when it is attempted to simultaneously obtain five variables (unknown quantity) consisting of four variables (unknown quantity) related to the cornea 14 and the glucose concentration $C_g$ by the least squares method or the like, it may be hard to obtain an accurate glucose concentration $C_g$ as illustrated in Fig. 8.

It is estimated on the ground that an optimum value is adjusted by a dominant (having a large contribution ratio) variable in a normal optimization calculation and thus the dominant variable (having a large contribution ratio) is accurately obtained, but a non-dominant (having a small contribution ratio) variable is affected by the dominant (having a large contribution ratio) variable and a large deviation of the non-dominant variable from the true value may easily occur.

In Example 1, as illustrated in Fig. 7, even in a case where five variables (unknown quantities) are simultaneously obtained, it is considered that when the initial value is set to be relatively near to the true value for each variable (unknown quantity), the glucose concentration $C_g$, which is the non-dominant variable, is also accurately obtained.

(Example 2)

In Example 2, the non-dominant (having a small contribution ratio) variable (here, glucose concentration $C_g$) is set as a temporary known quantity (constant), the least squares method is repeatedly performed for the dominant (having a large contribution ratio) variable (here, the variable related to the cornea 14), and the initial value, with which the objective function becomes smaller (to the minimum) than a case where other initial values are used for the objective function, is obtained. The initial is used as the adjusted initial value, the operation is performed again, and the non-dominant (having a small contribution ratio) variable (glucose concentration $C_g$) is obtained.

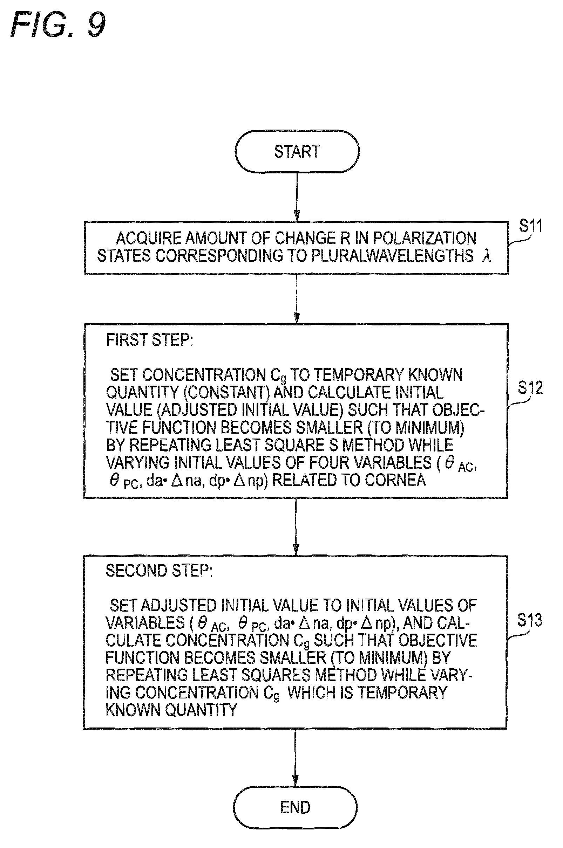

Fig. 9 is a flowchart for obtaining the result in Example 2.

The amount of change in the normalized polarization state R corresponding to the plural wavelengths $\lambda$ is acquired (Step S11. It is denoted by S11 in Fig. 9. Hereinafter, the same rule is applied).

Next, the glucose concentration $C_g$ is set as a temporary known quantity (constant), and the least squares method is repeated while varying four other unknown quantities (variables) from the initial value to thereby obtain (first step) initial values (adjusted initial values) of four unknown quantities (variable) in a case where the objective function of Equation (2) becomes smaller (to the minimum) than a case where other initial values are used for the objective function (Step S12).

The temporary known quantity of the glucose concentration $C_g$ is used as the initial value and the least squares method is repeated using four adjusted initial values while varying the adjusted initial values from the initial value to thereby obtain (second step) five values (results) of the variables (unknown quantities) in a case where the objective function of Equation (2) becomes smaller (to the minimum) than a case where other initial values are used for the objective function (Step S13).

That is, in Example 2, the glucose concentration $C_g$ is calculated by the first step where the glucose concentration $C_g$ is set as the temporary known quantity (constant) and the second step where the glucose concentration $C_g$ is

calculated by the adjusted initial value obtained in the first step. A calculation method in Example 2 may be denoted by a two-step method.

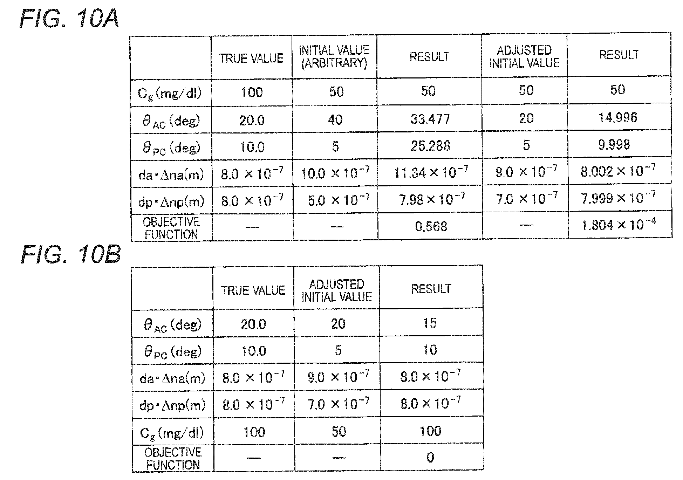

Fig. 10A and Fig. 10B are tables for illustrating results obtained by calculating the glucose concentration Cg in the two-step method. Fig. 10A illustrates a first step that calculates an adjusted initial value and Fig. 10B illustrates a second step that calculates the glucose concentration Cg using the adjusted initial value.

In the first step illustrated in Fig. 10A, regarding the glucose concentration Cg, a temporary known quantity (constant) is set as 50 mg/dl (third column) with respect to 100 mg/dl which is a true value (second column).

The least squares method is repeated while varying the initial values (arbitrary value) of four variables (unknown quantities) (here, the inclination $\theta_{AC}$ of the fast axis of the incidence side cornea 14A, the inclination $\theta_{PC}$ of the fast axis of the emission side cornea 14B, the product $da \cdot \Delta na$, and the product $dp \cdot \Delta np$) related to the cornea 14 to thereby obtain the initial value (adjusted initial value) of the variable (unknown quantity) in a case where the objective function becomes smaller (to the minimum) than a case where other initial values are used for the objective function.

In Fig. 10A, the true values are represented in the second column. The results obtained for initial values (third column) that are arbitrarily set are represented in the fourth column. When calculation is performed using the arbitrarily set initial values (third column), the objective function becomes 0.568.

The initial value is adjusted and the least squares method is repeated to thereby obtain the initial value with which the objective function becomes smaller (to the minimum) than a case where other initial values are used for the objective function.

In simulation, the initial values with which the objective function becomes smaller (to the minimum) are represented in the fifth column. The results obtained by the initial values are represented in the sixth column. In a case where the initial values are used, the objective function becomes $1.804 \times 10^{-4}$. The initial value is set as the adjusted initial value. The value of the objective function is much smaller than 0.568 obtained in a case where the initial values (third column) are arbitrarily set (third column).

In the simulation, the adjusted initial values correspond to the initial values in a case where the objective function becomes the minimum, are not necessarily the minimum, and may be values with which the objective function becomes smaller than a case where other initial values are used.

In the second step illustrated in Fig. 10B, the least squares method is repeated while varying 50 mg/dl, which is the glucose concentration Cg set as the temporary known quantity (constant), in the first step and the adjusted initial value obtained in Fig. 10A to thereby obtain values of the variables (unknown quantities) in a case where the objective function becomes smaller (to the minimum) than a case where other initial values are used for the objective function.

In Fig. 10B, the true values are represented in the second column, the adjusted initial values are represented in the third column, and the obtained results are represented in the fourth column. Here, when the objective function is 0 (minimum), and the glucose concentration Cg becomes 100 mg/dl, and coincides with the true value.

As described above, the glucose concentration Cg may be calculated by the two-step method to suppress an irrational value of the glucose concentration Cg from being calculated.

In the second step described above, the least squares method is repeated while varying the initial value and the adjusted initial value of the glucose concentration Cg as a variable to thereby calculate the glucose concentration Cg.

However, in a case where it is expected that an amount of change in the glucose concentration Cg is small or the like, the glucose concentration Cg may be varied (changed) in a stepwise manner, for example, from 50 mg/dl, 60 mg/dl, 70 mg/dl,... without setting the glucose concentration Cg as the variable. Even in such a case, when the glucose concentration $C_g$ is set to 100 mg/dl, the objective function becomes 0 and values illustrated in the results of Fig. 10B (fourth column) are obtained.

In the calculation of the concentration C of the optically active substance of the aqueous humor, the following method is considered as a method for excluding influence by the cornea 14.

First, a single wavelength $\lambda$ is used, a plural amounts of change in the polarization state (light intensity) obtained by allowing light to pass through the cornea 14 and the aqueous humor of the anterior eye chamber 13 are acquired by changing a rotation angle of the polarizer or the analyzer. The optical rotation degree $\phi_{AH}$, in which influence by the cornea 14 is excluded, is calculated by the least squares method on the basis of a theoretical formula, which includes a matrix representing the polarization property of the cornea 14 and a matrix representing the polarization property of the aqueous humor in the anterior eye chamber 13.

The calculation of the optical rotation degree $\phi_{AH}$ is conducted for light having different wavelengths $\lambda_i$ and calculates the optical rotation degree $\phi_{AHi}$ of the optically active substance which corresponds to each of the wavelengths $\lambda_i$, in which influence by the cornea 14 is excluded, and which is contained in the aqueous humor of in the anterior eye chamber 13.

Next, using a combination of the calculated wavelength $\lambda_i$ and the optical rotation degree $\phi_{AHi}$, the concentration C of the optically active substance is calculated by the least squares method on the basis of the theoretical formula which represents the optical rotation distribution of the optically active substance and in which the concentration C of the optically active substances is set as the unknown quantity.

However, in the method described above, it is required to individually conduct a step of calculating the optical rotation degree $\phi_{AH}$ and a step of calculating the concentration C of the optically active substance.

On the other hand, in the first exemplary embodiment, a matrix simply representing the polarization property of the aqueous humor in the anterior eye chamber 13 is not used as a matrix representing the polarization property of the aqueous humor, the polarization property is represented by a function of an expression representing the wavelength dependence of the optical rotation degree $\phi_{AH}$ of a specific optically active substance, and the expression contains the concentration C of the optically active substance as the unknown quantity or the temporary known quantity.

The operation is performed on the basis of the theoretical formula containing the matrix such that the concentration C of the optically active substance contained in the aqueous humor in the anterior eye chamber 13, in which influence by the cornea 14 is excluded, is directly calculated even without individually conducting the step of calculating the optical rotation degree $\phi_{AH}$ and the step of calculating the concentration C of the specific optically active substance.

Second Exemplary Embodiment

In the first exemplary embodiment, as illustrated in Fig. 2, light emitted from the eyeball 10 (the incidence side cornea 14A, the aqueous humor of the anterior eye chamber 13, the emission side cornea 14B) is separated into two linearly polarized lights that are orthogonal to each other by the analyzer 23 using the Wollaston prism or the like.

However, in a case where the light intensity is large and variation is small between the object to be measured 2 and the analyzer 23 of the optical system 20 of Fig. 2, the variable (unknown quantity) is calculated even without performing the normalization.

In the second exemplary embodiment, the concentration calculation system 1 of the optically active substance utilizes an analyzer 26 which does not separate light into two linearly polarized lights that are orthogonal to each other, instead of the analyzer 23 using the Wollaston prism or the like.

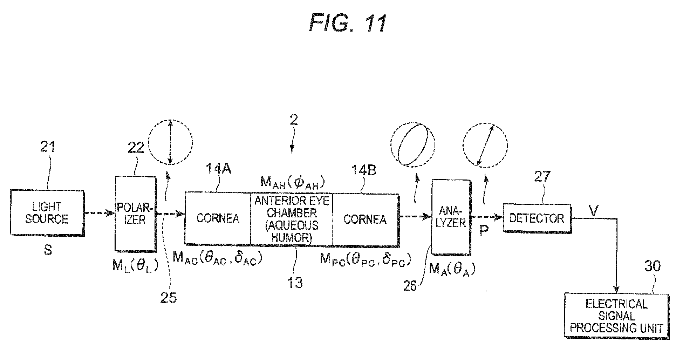

Fig. 11 is a diagram illustrating an outline of a concentration calculation system 1 of optically active substances according to a second exemplary embodiment. Here, the polarization separation type analyzer 23, which separates light into two linearly polarized lights that are orthogonal to each other, of the concentration calculation system 1 of the optically active substance according to the first exemplary embodiment illustrated in Fig. 2 is replaced with the analyzer 26 which does not separate light into two linearly polarized lights that are orthogonal to each other, and a single detector 27 is utilized for the analyzer 26.

The analyzer 26 is a total reflection type Glan-Thompson prism, a total reflection type Glan-Taylor prism, and a total reflection type Glan-Laser prism or the like, similar to the polarizer 22.

The detector 27 is a photo-detector such as a silicon diode similar to the detector 24A and the detector 24B, and outputs an electric signal corresponding to light intensity P of incident light. The detector 27 sets a voltage corresponding to the light intensity P of the incident light as a light intensity voltage V.

Other configurations of the second exemplary embodiment are the same as those of the first exemplary embodiment illustrated in Fig. 2 and the same constitutional elements are denoted by the same reference numerals and description thereof will not be repeated.

In a case where the object to be measured 2 is not present, the polarizer 22 and the analyzer 26 are arranged such that the linearly polarized light which passes through the analyzer 26 is orthogonal to the linearly polarized light which passes through the polarizer 22 (crossed-Nicol). In this case, the light intensity voltage V from the detector 27 is 0 in an ideal state.

The light intensity voltage V from the detector 27 corresponds to the amount of change in the polarization state generated by allowing light to pass through the cornea 14 and the aqueous humor of the anterior eye chamber 13, and is represented by Equation (17).

$$V = \left[M_A(\theta_A) \cdot M_{PC}(\theta_{PC}, \delta_{PC}) \cdot M_{AH}(\phi_{AH}) \cdot M_{AC}(\theta_{AC}, \delta_{AC}) \cdot M_L(\theta_L) \cdot S\right]_0 \qquad \cdots (17)$$

That is, the least squares method may be applied using Equation (17) instead of Equation (10) in the first exemplary embodiment.

As described above, the analyzer 26 which is not a polarization separation type analyzer is utilized and thus a configuration of the optical system 20 becomes simple and an optical axis adjustment becomes easy.

Third Exemplary Embodiment

In the first exemplary embodiment, as illustrated in Fig. 2, light is separated into two linearly polarized lights that are orthogonal to each other by the polarization separation type analyzer 23 and the polarization is normalized using a sum ($V_A$ + $V_B$) of the light intensity voltage $V_A$ and the light intensity voltage $V_B$ of the two linearly polarized lights (see Equation (1)). As described above, the sum ($V_A$ + $V_B$) of the light intensity voltage $V_A$ and the light intensity voltage $V_B$ corresponds to the light intensity before light is incident to the analyzer 23.

In the third exemplary embodiment, light is split between the object to be measured 2 and the analyzer 23 of the optical system 20 in Fig. 2, instead of separating light into two linearly polarized lights by the polarization separation type analyzer 23.

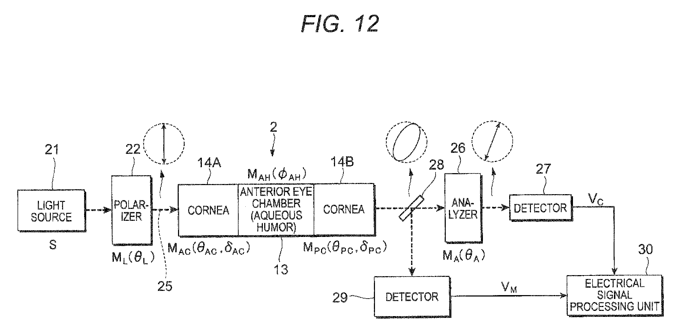

Fig. 12 is a diagram illustrating an outline of a concentration calculation system 1 of the optically active substance according to a third exemplary embodiment. Here, similar to the concentration calculation system 1 of the optically active substance according to the second exemplary embodiment illustrated in Fig. 11, the analyzer 26, which is not a polarization separation type analyzer, is utilized and a single detector 27 is utilized for the analyzer 26.

Furthermore, an optical splitting member 28 splitting light and a detector 29 detecting light split by the optical splitting member 28 are provided between the object to be measured 2 and the analyzer 26 of the optical system 20 in the concentration calculation system 1 of the optically active substance according to the third exemplary embodiment.

The optical splitting member 28 is a half mirror, which allows a portion of incident light to pass through and reflects the remaining incident light, or the like.

The detector 29 is a photo-detector such as a silicon diode similar to the detector 27, and outputs an electric signal corresponding to light intensity of incident light. The detector 29 outputs a light intensity voltage $V_M$.

The detector 27 outputs the light intensity voltage $V_C$. The light intensity voltage $V_C$ corresponds to a voltage obtained by multiplying the light intensity voltage $V$ output from the detector 27 in the second exemplary embodiment by a transmittance T of the optical splitting member 28. Here, it is regarded that the optical splitting member 28 splits light without loss and reflectance becomes (1 - T) due to transmittance of T. It is regarded that the optical splitting member 28 does not influence the polarization state at all.

In a case where the object to be measured 2 is not present, the polarizer 22 and the analyzer 26 are arranged such that the linearly polarized light which passes through the analyzer 26 is orthogonal to the linearly polarized light which passes through the polarizer 22 (crossed-Nicol). In this case, the light intensity voltage $V_C$ from the detector 27 is 0 in an ideal state.

The normalized light intensity N can be obtained by dividing the light intensity voltage $V_C$ by the light intensity voltage $V_M$ from the detector 29. The normalized light intensity N is represented by Equation (18).

It is possible to suppress influence due to variation of the light intensity of light emitted by the light source 21 using the normalized light intensity N.

$$N = \frac{V_C}{V_M} = \frac{\left[M_A(\theta_A) \cdot T \cdot M_{PC}(\theta_{PC}, \delta_{PC}) \cdot M_{AH}(\phi_{AH}) \cdot M_{AC}(\theta_{AC}, \delta_{AC}) \cdot M_L(\theta_L) \cdot S\right]_0}{\left[(1-T) \cdot M_{PC}(\theta_{PC}, \delta_{PC}) \cdot M_{AH}(\phi_{AH}) \cdot M_{AC}(\theta_{AC}, \delta_{AC}) \cdot M_L(\theta_L) \cdot S\right]_0} \quad \cdots (1 8)$$

As described above, the analyzer 26 which is not a polarization separation type analyzer is utilized and thus a configuration of the optical system 20 becomes simple and an optical axis adjustment becomes easy. It is possible to suppress influence due to variation of the light intensity of light emitted by the light source 21 using the normalized light intensity N.

From the first exemplary embodiment to the third exemplary embodiment, the Mueller matrix M and the Stokes vector S are used to represent the polarization state. The Jones matrix J which represents the polarization state using the electric field and the Jones vector E may be used instead of the Mueller matrix M and the Stokes vector S.

The conversion of the equation may be performed to be similarly applied.

From the first exemplary embodiment to the third exemplary embodiment, although the least squares method is applied, a value of a function (an amount of change in the normalized polarization state R or the like) of the wavelengths $\lambda$, of which the number is the same as that of the unknown quantities, may be used so as to calculate the concentration C of the optically active substance which is the unknown quantity.

Furthermore, in the concentration calculation system 1 of the optically active substance according to the first exemplary embodiment to the third exemplary embodiment, light is allowed to pass through the aqueous humor of the anterior eye chamber 13 through the cornea 14 (incidence side cornea 14A and emission side cornea 14B) of the eyeball 10 to calculate the concentration (glucose concentration) Cg of glucose which is the optically active substance contained in the aqueous humor.

The concentration calculation system 1 of the optically active substance may be applied to the calculation of the concentration of other optically active substances contained in the aqueous humor. Furthermore, it is possible to calculate concentrations of plural optically active substances contained in the aqueous humor by separating the plural optically active substances.

The concentration calculation system 1 of the optically active substance may be applied to a case where light is allowed to pass through plural members having an optical property such as birefringence or optical activity or the like to calculate the concentration of the optically active substance contained.

**Claims**

1. A concentration calculation system of an optically active substance, comprising:

   a calculation unit configured to:

   acquire an amount of change in a polarization state by allowing light having different wavelengths to pass through a cornea and an aqueous humor; and
   calculate a concentration of a specific optically active substance contained in the aqueous humor by a least squares method based on a theoretical formula which includes a matrix representing a polarization property of the cornea and a matrix representing a polarization property of the aqueous humor and represents a wavelength dependence of the amount of change in the polarization state,

   wherein the matrix representing the polarization property of the aqueous humor is represented by a function of an expression representing the wavelength dependence of an optical rotation degree of the specific optically active substance and the expression includes a concentration value of the specific optically active substance as an unknown quantity or a temporal known quantity.

2. The concentration calculation system of an optically active substance according to claim 1,

   wherein the calculation unit is configured to calculate the concentration of the specific optically active substance by:

   setting the value of the concentration of the specific optically active substance contained in the aqueous humor to the temporary known quantity and obtaining an initial value adjusted with regard to the cornea by the least squares method such that an objective function, that is a sum of squares of a difference between the acquired amount of change in the polarization state and the theoretical formula becomes smaller than a case where other initial values are used for the objective function; and
   calculating values allocated to the unknown quantities related to the aqueous humor and the cornea in the least squares method using the adjusted initial value such that the objective function becomes smaller than a case where other values are allocated for the objective function.

3. The concentration calculation system of an optically active substance according to claim 2,

   wherein the calculating calculates values allocated to the unknown quantities related to the aqueous humor and the cornea such that the objective function becomes smaller than the case where other values are allocated for the objective function by varying the values in a stepwise manner as the concentration of the specific optically active substance.

4. A concentration calculation system of an optically active substance, comprising:

   a calculation unit configured to:

   acquire an amount of change in a polarization state by allowing light having different wavelengths to pass through a cornea and an aqueous humor; and
   calculate a concentration of a specific optically active substance contained in the aqueous humor by solving a simultaneous equation configured with equations of same number as the number of unknown quantities included in a theoretical formula based on the theoretical formula which includes a matrix representing a polarization property of the cornea and a matrix representing a polarization property of the aqueous humor and represents a wavelength dependence of the amount of change in the polarization state,

   wherein the matrix representing the polarization property of the aqueous humor is represented by a function of an expression representing the wavelength dependence of an optical rotation degree of the specific optically active substance and the expression includes a concentration value of the specific optically active substance as an unknown quantity.

5. A program causing a computer to execute a process for calculating a concentration of an optically active substance, the process comprising:

acquiring an amount of change in a polarization state by allowing light having different wavelengths to pass through a cornea and an aqueous humor; and
calculating a concentration of a specific optically active substance contained in the aqueous humor by a least squares method on the basis of a theoretical formula which includes a matrix representing a polarization property of the cornea and a matrix representing a polarization property of the aqueous humor and represents a wavelength dependence of the amount of change in the polarization state,
wherein the matrix representing the polarization property of the aqueous humor is represented by a function of an expression representing the wavelength dependence of an optical rotation degree of the specific optically active substance and the expression includes a concentration value of the specific optically active substance as an unknown quantity or a temporal known quantity.

6. A program causing a computer to execute a process for calculating a concentration of an optically active substance, the process comprising:

acquiring an amount of change in a polarization state by allowing light having different wavelengths to pass through a cornea and an aqueous humor; and
calculating a concentration of a specific optically active substance contained in the aqueous humor by solving a simultaneous equation configured with equations of same number as the number of unknown quantities included in a theoretical formula based on the theoretical formula which includes a matrix representing a polarization property of the cornea and a matrix representing a polarization property of the aqueous humor and represents a wavelength dependence of the amount of change in the polarization state,
wherein the matrix representing the polarization property of the aqueous humor is represented by a function of an expression representing the wavelength dependence of an optical rotation degree of the specific optically active substance and the expression includes a concentration value of the specific optically active substance as an unknown quantity.

## FIG. 1A

10
11
12
16

REAR SIDE

FRONT SIDE

NOSE

14B
13 15 14
14A
25

INTERNAL CANTHUS
SIDE (NOSE-SIDE)

OUTER CANTHUS
SIDE (EAR-SIDE)

## FIG. 1B

13
10

UPPER SIDE

LOWER SIDE

25

15

INTERNAL CANTHUS
SIDE (NOSE-SIDE)

OUTER CANTHUS
SIDE (EAR-SIDE)

EP 3 120 768 A1

# FIG. 2

20

*FIG. 3*

$$R = \frac{V_A - V_B}{V_A + V_B}$$

# FIG. 4

# FIG. 5

## FIG. 6

$$R = \frac{V_A - V_B}{V_A + V_B}$$

# FIG. 7

|  | TRUE VALUE | INITIAL VALUE | RESULT |
|---|---|---|---|
| $\theta_{AC}$ (deg) | 20.0 | 20 | 15 |
| $\theta_{PC}$ (deg) | 10.0 | 5 | 10 |
| da·$\Delta$na(m) | $8.0 \times 10^{-7}$ | $9.0 \times 10^{-7}$ | $8.0 \times 10^{-7}$ |
| dp·$\Delta$np(m) | $8.0 \times 10^{-7}$ | $7.0 \times 10^{-7}$ | $8.0 \times 10^{-7}$ |
| $C_g$ (mg/dl) | 100 | 50 | 100 |
| OBJECTIVE FUNCTION | — | — | 0 |

## FIG. 8

| | TRUE VALUE | INITIAL VALUE (ARBITRARY) | RESULT | RESULT ( CONSTRAINTS $C_g > 0$ ) |
|---|---|---|---|---|
| $\theta_{AC}$ (deg) | 20.0 | 40 | 14.265 | 20.011 |
| $\theta_{PC}$ (deg) | 10.0 | 5 | 10.689 | 9.997 |
| da·$\Delta$na(m) | $8.0 \times 10^{-7}$ | $10.0 \times 10^{-7}$ | $10.77 \times 10^{-7}$ | $7.998 \times 10^{-7}$ |
| dp·$\Delta$np(m) | $8.0 \times 10^{-7}$ | $5.0 \times 10^{-7}$ | $4.858 \times 10^{-7}$ | $8.003 \times 10^{-7}$ |
| $C_g$ (mg/dl) | 100 | .50 | −360642 | 0 |
| OBJECTIVE FUNCTION | — | — | 0.021 | $1.804 \times 10^{-4}$ |

# FIG. 9

START

ACQUIRE AMOUNT OF CHANGE R IN POLARIZATION STATES CORRESPONDING TO PLURALWAVELENGTHS $\lambda$ — S11

FIRST STEP:

SET CONCENTRATION $C_g$ TO TEMPORARY KNOWN QUANTITY (CONSTANT) AND CALCULATE INITIAL VALUE (ADJUSTED INITIAL VALUE) SUCH THAT OBJECTIVE FUNCTION BECOMES SMALLER (TO MINIMUM) BY REPEATING LEAST SQUARE S METHOD WHILE VARYING INITIAL VALUES OF FOUR VARIABLES ($\theta_{AC}$, $\theta_{PC}$, da·$\triangle$na, dp·$\triangle$np) RELATED TO CORNEA — S12

SECOND STEP:

SET ADJUSTED INITIAL VALUE TO INITIAL VALUES OF VARIABLES ($\theta_{AC}$, $\theta_{PC}$, da·$\triangle$na, dp·$\triangle$np), AND CALCULATE CONCENTRATION $C_g$ SUCH THAT OBJECTIVE FUNCTION BECOMES SMALLER (TO MINIMUM) BY REPEATING LEAST SQUARES METHOD WHILE VARYING CONCENTRATION $C_g$ WHICH IS TEMPORARY KNOWN QUANTITY — S13

END

FIG. 10A

| | TRUE VALUE | INITIAL VALUE (ARBITRARY) | RESULT | ADJUSTED INITIAL VALUE | RESULT |
|---|---|---|---|---|---|
| $C_g$ (mg/dl) | 100 | 50 | 50 | 50 | 50 |
| $\theta_{AC}$ (deg) | 20.0 | 40 | 33.477 | 20 | 14.996 |
| $\theta_{PC}$ (deg) | 10.0 | 5 | 25.288 | 5 | 9.998 |
| da·$\Delta$na(m) | $8.0 \times 10^{-7}$ | $10.0 \times 10^{-7}$ | $11.34 \times 10^{-7}$ | $9.0 \times 10^{-7}$ | $8.002 \times 10^{-7}$ |
| dp·$\Delta$np(m) | $8.0 \times 10^{-7}$ | $5.0 \times 10^{-7}$ | $7.98 \times 10^{-7}$ | $7.0 \times 10^{-7}$ | $7.999 \times 10^{-7}$ |
| OBJECTIVE FUNCTION | — | — | 0.568 | — | $1.804 \times 10^{-4}$ |

FIG. 10B

| | TRUE VALUE | ADJUSTED INITIAL VALUE | RESULT |
|---|---|---|---|
| $\theta_{AC}$ (deg) | 20.0 | 20 | 15 |
| $\theta_{PC}$ (deg) | 10.0 | 5 | 10 |
| da·$\Delta$na(m) | $8.0 \times 10^{-7}$ | $9.0 \times 10^{-7}$ | $8.0 \times 10^{-7}$ |
| dp·$\Delta$np(m) | $8.0 \times 10^{-7}$ | $7.0 \times 10^{-7}$ | $8.0 \times 10^{-7}$ |
| $C_g$ (mg/dl) | 100 | 50 | 100 |
| OBJECTIVE FUNCTION | — | — | 0 |

FIG. 11

LIGHT SOURCE 21

POLAR-IZER 22

CORNEA 14A

ANTERIOR EYE CHAMBER (AQUEOUS HUMOR)

CORNEA 14B

ANA-LYZER 26

DETECTOR 27

ELECTRICAL SIGNAL PROCESSING UNIT 30

$S$

$M_L(\theta_L)$

$M_{AC}(\theta_{AC}, \delta_{AC})$

$M_{AH}(\phi_{AH})$

$M_{PC}(\theta_{PC}, \delta_{PC})$

$M_A(\theta_A)$

$P$

$V$

25

13

2

*FIG. 12*

Europäisches
Patentamt

European
Patent Office

Office européen
des brevets

# EUROPEAN SEARCH REPORT

**Application Number**

EP 16 17 8509

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/225321 A1 (COTE GERARD L [US] ET AL) 4 December 2003 (2003-12-04) * paragraphs [0008], [0009], [0066] - [0074] * * figure 1 * | 1-6 | INV. A61B5/145 A61B5/1455 |
| X | DE 10 2008 013821 A1 (WESTPHAL PETER [DE]) 17 September 2009 (2009-09-17) * paragraphs [0015], [0017], [0032] - [0038] * * figures 1,2,4,6-8 * | 1-6 | |
| X | DE 10 2005 020912 A1 (ZEISS CARL MEDITEC AG [DE]; ZEISS CARL JENA GMBH [DE]) 30 November 2006 (2006-11-30) * paragraphs [0007], [0037], [0054] - [0062], [0067] - [0073], [0076] - [0081] * * figure 3 * | 1-6 | |

| | | | TECHNICAL FIELDS SEARCHED (IPC) |
|---|---|---|---|
| | | | A61B |

The present search report has been drawn up for all claims

| Place of search | Date of completion of the search | Examiner |
|---|---|---|
| Munich | 12 December 2016 | Olapinski, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 17 8509

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2016

| Patent document cited in search report | Publication date | Patent family member(s) | Publication date |
|---|---|---|---|
| US 2003225321 A1 | 04-12-2003 | NONE | |
| DE 102008013821 A1 | 17-09-2009 | DE 102008013821 A1<br>DE 102009040854 A1<br>EP 2252200 A1<br>US 2011105868 A1<br>US 2014249392 A1<br>WO 2009112009 A1 | 17-09-2009<br>10-03-2011<br>24-11-2010<br>05-05-2011<br>04-09-2014<br>17-09-2009 |
| DE 102005020912 A1 | 30-11-2006 | NONE | |

EPO FORM P0459

For more details about this annex : see Official Journal of the European Patent Office, No. 12/82

**EP 3 120 768 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008032594 A
- JP 2007139722 A
- JP 2012103222 A
- JP 2014130045 A
- JP 2007111159 A